# EUROPÄISCHE PATENTSCHRIFT

(11) **EP 1 789 444 B1**
(45) Veröffentlichungstag und Bekanntmachung des Hinweises auf die Patenterteilung: **14.10.2015**
(21) Anmeldenummer: 05791971.4
(22) Anmeldetag: 09.09.2005
(51) Int. Cl.: C07K 14/745

(54) **HERSTELLUNG EINES VON WILLEBRAND FAKTOR-PRÄPARATES UNTER VERWENDUNG VON HYDROXYLAPATIT**
PRODUCTION OF A VON WILLEBRAND FACTOR PREPARATION USING HYDROXYLAPATITE
PRODUCTION D'UNE PREPARATION DE FACTEUR WILLEBRAND AU MOYEN D'HYDROXYLAPATITE

(30) Priorität: 14.09.2004 DE 102004044419
(43) Veröffentlichungstag der Anmeldung: 30.05.2007
(73) Patentinhaber: Biotest AG, D-63303 Dreieich (DE)
(72) Erfinder: KRETSCHMAR, Michael, 63500 Seligenstadt (DE); MÖLLER, Wolfgang, 61440 Oberursel (DE)
(74) Vertreter: Kalhammer, Georg
(86) Internationale Anmeldenummer: PCT/EP2005/009728
(87) Internationale Veröffentlichungsnummer: WO 2006/029773

(56) Entgegenhaltungen:
- US-A- 5 128 245
- FEDERICI AUGUSTO B: "The factor VIII/von Willebrand factor complex: basic and clinical issues." HAEMATOLOGICA, Bd. 88, Nr. 6, Mai 2003 (2003-05), Seiten 3-12, XP002356462 ISSN: 1592-8721
- GORMAN J J ET AL: "STUDIES ON THE STRUCTURE AND SUBUNIT COMPOSITION OF HUMAN ANTI HEMOPHILIC FACTOR" THROMBOSIS RESEARCH, Bd. 12, Nr. 2, 1978, Seiten 341-352, XP002356463 ISSN: 0049-3848
- SAUNDRY R H ET AL: "CHROMATOGRAPHY OF VWF ON DEXTRAN SULFATE SEPHAROSE" THROMBOSIS RESEARCH, Bd. 48, Nr. 6, 1987, Seiten 641-652, XP002356464 ISSN: 0049-3848
- JANSON JC, RYDEN L (EDTS): "Protein purification-principles, high-resolution methods and applications" 1998, WILEY-LISS , NEW YORK , XP002356473 Seite 190, Absatz 6 - Seite 191, Absatz 6 Seite 199; Abbildung 4.14 Seite 200, Absatz 1 - Absatz 2
- BERNARDI G: "Chromatography of proteins on hydroxyapatite" METHODS IN ENZYMOLOGY, Bd. 27, 1973, Seiten 471-479, XP009057844

## Beschreibung

Die Erfindung betrifft ein Verfahren zur Aufreinigung von von Willebrand Faktor, wobei wenigstens eine Hydroxylapatit-Durchlaufchromatographie durchgeführt wird, bei der VWF im wesentlichen nicht gebunden wird, während Verunreinigungsproteine an das Chromatographiemedium gebunden werden..

Der von Willebrand Faktor ist ein Glykoprotein, das in Endothelzellen und Megakaryozyten synthetisiert wird. Das Molekulargewicht des Monomers beträgt ca. 225 000 Da. Durch Ausbildung von Disulfidbrücken werden in der Zelle Dimere ausgebildet, die wiederum zu Oligomeren aus bis zu 40 dimeren Untereinheiten ebenfalls über Disulfidverknüpfungen assoziieren. Die Konzentration des in das Plasma abgegebenen von Willebrand Faktors (VWF) beträgt 5 - 10 mg/l.

In der primären Hämostase hat der VWF die Aufgabe, die Adhäsion von Thrombozyten an verletztes Subendothel zu vermitteln und unter Bedingungen hoher Scherkräfte, wie im arteriellen System, die Thrombozytenaggregation zu unterstützen. Als Funktion in der sekundären Hämostase bindet VWF Faktor VIII, einen wichtigen Cofaktor bei der Blutgerinnung, in einem nicht kovalenten Komplex. Faktor VIII wird dadurch stabilisiert und vor vorzeitigem Abbau geschützt.

Das von Willebrand Syndrom (VWS) ist eine Bluterkrankheit, die durch quantitative oder qualitative Veränderung des VWF hervorgerufen wird. Mit einer Prävalenz von 0,8 - 1,3% ist das VWS die häufigste vererbbare Bluterkrankheit. Sie trifft, im Gegensatz zu Hämophilie A, Männer und Frauen gleichermaßen. Das VWS wird in verschiedene Typen klassifiziert: Typ 1-Patienten weisen einen niedrigeren VWF-Spiegel im Blut auf. Unter dem VWS Typ 2 sind sämtliche qualitativen Defekte des VWF im Plasma zusammengefasst. Störungen können in der primären und/oder sekundären Hämostase auftreten. Bei Typ 3-Patienten fehlt der VWF komplett. Einige Untertypen des Typ 2 und der Typ 3 werden als schwere Form des VWS bezeichnet. Bei der schweren Form des VWS sind Ersatztherapien mit VWF-haltigen Präparaten notwendig.

Häufig werden für die Behandlung der schweren Form des VWS plasmatische Faktor VIII-Präparate eingesetzt, die einen relativ höhen Anteil an VWF enthalten. Blutungen können damit zwar gestillt werden, gleichzeitig steigt aber, besonders bei häufiger oder längerer Anwendung, das Thromboserisiko. Dies ist auf eine Überdosierung von Faktor VIII zurückzuführen. Wesentlich günstiger im Sinne der Patientensicherheit ist die Therapie von VWS-Kranken mit hochreinen, Faktor VIII-armen oder -freien VWF-Konzentraten.

In der Literatur sind verschiedene Verfahren zur Reinigung und Gewinnung von VWF beschrieben worden:

Die EP-A-0 416 983 offenbart die Gewinnung eines VWF-Faktor VIII-Komplexes aus menschlichem Plasma durch Ausfällung mit einer Kombination aus Bariumchlorid und Aluminiumhydroxid und anschließender Anionenaustauschchromatographie an DEAE-Fraktogel.

EP 0 469 985 stellt ein Verfahren dar, bei dem VWF aus Kryopräzipitat unter Verwendung eines Anionenaustauschers gewonnen wird. Dabei wird Faktor VIII bei einer Salzkonzentration von 250 mM gebunden, während VWF im Überstand verbleibt. Der Überstand wird nach Erniedrigung der Salzkonzentration auf 100 - 150 mM an einen zweiten Anionenaustauscher gebunden und mit 300 - 350 mM NaCl eluiert.

In der EP 0 503 991 wird die Reinigung von VWF aus humanem Kryopräzipitat durch einen 3-stufigen Prozess beschrieben: 1. Anionenaustauschchromatographie mit DEAE-Fraktogel und Elution des VWF durch 0,15 M NaCl. 2. Rechromatographie an DEAE-Fraktogel und Elution des VWF durch 0,17 M NaCl. 3. Abtrennung von Fibronektin und Fibrinogen durch Gelatine-Sepharose-Affinitätschromatographie. VWF befindet sich dabei im Durchlauf. Als Puffer werden Aminosäuren und Calciumionen enthaltende Lösungen verwendet.

Die WO 89/12065 beschreibt die Trennung von Plasma-Proteinen aus Plasma-Kryopräzipitaten durch Bindung der Proteine an DEAE-Fraktogel und stufenweise Elution durch steigende Zugabe von NaCl. Das Verfahren eignet sich insbesondere zur Gewinnung von Faktor VIII-Konzentraten, sowie zur Darstellung von Konzentraten von Fibrinogen, Fibronektin und VWF.

Die WO 96/10584 beschreibt ein Verfahren zur Gewinnung von hochreinem rekombinanten VWF mittels kombinierter Anionenaustausch-/Heparin-Affinitätschromatographie und die EP 0 705 846 die Trennung von hoch- und niedermolekularen Fraktionen von rekombinantem VWF mittels Heparin-Affinitätschromatographie.

WO 98/38219 beschreibt ein Verfahren zur Gewinnung von VWF, bei dem der VWF bei einer niedrigen Salzkonzentration an einen Kationenaustauscher gebunden wird und durch fraktionierte Elution VWF mit hoher spezifischer Aktivität gewonnen wird.

In "The factor VIII / von Willebrand factor complex: basic and clinical issues", Augusto B. Federici, Haematologica vol. 88 (supplement 9) werden Zusammensetzungen, die VWF enthalten beschrieben.

US 5,128,245 beschreibt die Verwendung von Hydroxyapatit zur Aufreinigung von VWF.

In "studies on the structure and subunit composition of human antihaemophilic factor", J. J. Gorman, Thrombosis Research, vol. 12, Nr. 2, 1978 wird ein Verfahren zur Aufreinigung des Faktor VIII-VWF Komplexes durch Bindung des Komplexes an Hydroxylapatit beschrieben.

In "Chromatography of vWF on dextran sulphate sepharose", R.H. Saundry, Thrombosis research vol. 48, Nr. 6, 1987 wird ein Verfahren zur Aufreinigung von VWF mit Hilfe von Dextransulfat Agarose beschrieben.

Die bisher beschriebenen Verfahren sind mit Nachteilen behaftet. Eine AnionenaustauschChromatographie weist eine unbefriedigende Auflösung auf. Für die Gewinnung eines hochreinen Präparates ist in der Regel eine Kombination mit einer Affinitätschromatographie notwendig. Affinitätschromatographien wiederum sind kostenintensiv. In der Kombination von Anionenaustausch- und KationenaustauschChromatographie wurden für ein plasmatische VWF-Präparat spezifische Aktivitäten von maximal 83 E/mg Protein erzielt. Ebenso wurde bei einem Verfahren, bei dem für die Aufreinigung eines rekombinanten VWF Anionenaustausch-, Immunoaffinitäts- und Kationenaustauschchromatographie verwendet wurden, eine spezifische Aktivität von nur 59,2 E/mg Protein erzielt.

Eine Aufgabe der vorliegenden Erfindung ist es, ein einfaches, effektives Herstellverfahren zur Verfügung zu stellen, mit dem ein hochreines, im wesentlichen Faktor VIII-freies VWF-Präparat mit einer hohen spezifischen Aktivität kostengünstig hergestellt werden kann.

Überraschenderweise wurde gefunden, dass ein Chromatographieschritt mit Hydroxylapatit als Chromatographie-Matrix vorteilhafte Ergebnisse zeigt. Es wurde überraschenderweise festgestellt, dass Hydroxylapatit nicht nur als bindende Chromatographie, bei der VWF gebunden wird und die Verunreinigungen ungebunden bleiben bzw. in einer anderen Stufe eluiert werden, sondern auch als Durchlaufchromatographie, bei der VWF ungebunden durch die Säule läuft und Verunreinigungen gebunden werden, verwendet werden kann.

Erfindungsgemäß wird daher Hydroxylapatit als Durchlaufchromatographie-Medium zur Aufreinigung des VWF verwendet. Die vorliegende Erfindung betrifft ein Verfahren zur Aufreinigung von VWF, dadurch gekennzeichnet, dass wenigstens eine Hydroxylapatit-Chromatographie, bei der VWF im wesentlichen nicht gebunden wird, während Verunreinigungsproteine an das Chromatographiemedium absorbiert werden, durchgeführt wird.

Vorzugsweise binden weniger als 30 %, bevorzugter weniger als 25 %, noch bevorzugter weniger als 20 %, am bevorzugtesten weniger als 10 % des in der Auftragslösung vorhandenen VWF an die Hydroxylapatitmatrix.

Hydroxylapatit ist eine Form von Calciumphosphat mit der Zusammensetzung Ca₅(PO₄)₃OH bzw. Ca₁₀(PO₄)₆OH₂, die als stationäre Phase für die Chromatographie von Proteinen, Nukleinsäuren und anderen Makromolekülen eingesetzt werden kann. Neben der kristallinen Form von Hydroxylapatit kann auch eine keramische Form verwendet werden, welche durch Sintern erhältlich ist. Hydroxylapatit kann beispielsweise von der Firma Bio-Rad (München, Deutschland) bezogen werden. Deren keramisches Hydroxylapatit wird in zwei Formen (Typ 1 und Typ 2) zur Verfügung gestellt. Typ 1-Material hat aufgrund größerer Oberflächen eine höhere Bindungskapazität für kleinere Moleküle, z.B. kleine Proteine. Dagegen weist das Typ 2-Material größere Poren in den Partikeln auf, die ein Eindringen und damit eine bessere Bindung von großen Molekülen, z.B. DNA oder großen Proteinen, ermöglicht. Die Materialien weisen vorzugsweise folgende Eigenschaften auf:

**Tabelle 1**

| | Dynamische Bindungskapazität | Nominale Porendurchmesser |
|---|---|---|
| Typ 1 | >13,7 mg Lysozym/ml CHT* | 600-800 Å |
| Typ 2 | >6,8 mg Lysozym/ml CHT* | 800-1000 Å |

| | | |
|---|---|---|
| *CHT=Ceramic Hydroxy Apatite | | |

Kristallines oder keramisches Hydroxylapatit ist frei verfügbar. Verfahren zu ihrer Herstellung sind im Stand der Technik bekannt.

Das erfindungsgemäße Verfahren beinhaltet, dass (i) eine Zusammensetzung, die VWF und ein oder mehrere verunreinigende Proteine enthält, mit einer Hydroxylapatit-Matrix in Kontakt gebracht wird, so dass wenigstens ein verunreinigendes Protein an die Hydroxylapatit-Matrix gebunden wird, während VWF im wesentlichen nicht an die Hydroxylapatit-Matrix gebunden wird, und gegebenenfalls anschließend (ii) ungebundenes VWF von der Hydroxylapatit-Matrix getrennt wird. Diese Ausführungsform wird in der vorliegenden Anmeldung als "Durchlaufchromatographie" bezeichnet.

Das Verfahren kann in Form einer Säulenchromatographie oder im Batch-Verfahren durchgeführt werden; die Durchführung einer Säulenchromatographie ist bevorzugt. Im Fall einer Säulenchromatographie befindet sich VWF im Durchlauf und wenigstens ein verunreinigendes Protein, z. B. Fibronektin und/oder Fibrinogen, wird an das Hydroxylapatit gebunden.
Die Hydroxylapatit-Chromatographie wird gemäß dieser Ausführungsform bei einem pH-Wert von 6,5 bis 8,5, vorzugsweise von 6,8 bis 8,5, bevorzugter von 6,8 bis 7,5, am bevorzugtesten von 7,0 bis 7,5 durchgeführt. Üblicherweise haben Lauf-, Wasch- und Elutionspuffer, sowie die aufzutragende Proteinlösung den gleichen pH-Wert. Es sind aber auch Varianten praktikabel, bei denen diese Lösungen unterschiedliche pH-Werte aufweisen.

Die VWF enthaltende Zusammensetzung, die mit der Hydroxylapatitmatrix in Kontakt gebracht wird, enthält vorzugsweise Natriumphosphat und/oder Kaliumphosphat. Die Gesamtkonzentration an Natriumphosphat und/oder Kaliumphosphat in der Lösung ist beispielsweise 0 bis 100 mM, vorzugsweise 10 bis 50 mM, am bevorzugtesten 20 bis 40 mM, d. h. als Laufpuffer kann eine Pufferlösung mit den genannten Konzentrationen eingesetzt werden.

Die VWF-haltige Lösung, z.B. eine vorgereinigte Kryopräzipitatlösung, wird bei einer niedrigen Salzkonzentration von 0 - 100 mM Kalium- oder Natriumphosphat, bevorzugt bei 10 - 50 mM, bei einem pH von vorzugsweise 6,8 bis 8,5, besonders bevorzugt bei einem pH von 7,0 - 7,5, auf eine Hydroxylapatit-Säule aufgetragen. Das Hydroxylapatit ist in dieser Ausführungsform vorzugsweise keramisches Hydroxylapatit, besonders bevorzugt vom Typ 1, wie von Bio-Rad (München, Deutschland) vertrieben. Unter diesen Bedingungen bindet der Großteil der VWF-Moleküle nicht an die Matrix und befindet sich im Durchlauf, während Verunreinigungsproteine, wie z.B. Fibrinogen oder Fibronektin, zum Großteil an die Matrix binden.

Durch die erfindungsgemäße Durchlaufchromatographie können VWF-Präparationen erhalten werden, die nur geringe Mengen an Fibrinogen und Fibronektin enthalten. In der Regel ist die Konzentration an Fibrinogen-Antigen in der Durchlauffraktion niedriger als 25 µg/ml, bevorzugt niedriger als 15 µg/ml, bevorzugter niedriger als 10 µg/ml, am bevorzugtesten höchstens 5 µg/ml. Die Konzentration an Fibronektin-Antigen in der Durchlauffraktion ist üblicherweise niedriger als 250 µg/ml, bevorzugt niedriger als 150 µg/ml, bevorzugter niedriger als 100 µg/ml, am bevorzugtesten höchstens 50 µg/ml. Die Konzentration an Fibrinogen-Antigen und Fibronektin-Antigen kann durch an sich bekannte Verfahren bestimmt werden, z. B. wie in den Beispielen der vorliegenden Anmeldung beschrieben. Die Abreicherung von Fibronektin und Fibrinogen empfiehlt sich insbesondere deswegen, weil diese Proteine aufgrund ihrer Aggregationsneigung prozesstechnische Probleme, z.B. bei Filtrationen verursachen können. Fibronektin und Fibrinogen in lyophylisierten Endprodukten verhindern häufig die vollständige Lösbarkeit eines Präparates.

Wenn die Auftragslösung, die mit der Hydroxylapatit-Matrix in Kontakt gebracht wird, Fibrinogen und/oder Fibronektin enthält (z. B. weil es sich um eine Plasmafraktion handelt), kann eine erhebliche Abreicherung der verunreinigenden Proteine Fibrinogen und Fibronektin erzielt werden. So ist die Konzentration an Fibrinogen in der Durchlauffraktion vorzugsweise niedriger als 10%, bevorzugter niedriger als 5%, noch bevorzugter niedriger als 2,5% der Konzentration an Fibrinogen in der Auftragslösung (vor Durchlaufchromatographie). Die Konzentration an Fibronektin in der Durchlauffraktion ist vorzugsweise niedriger als 10%, bevorzugter niedriger als 5%, noch bevorzugter niedriger als 2,5% der Konzentration an Fibronektin in der Auftragslösung (vor Durchlaufchromatographie).

Die Ausbeute der Durchlaufchromatographie (bezogen auf die Massenbilanz) ist in der Regel höher als 50%, bevorzugt höher als 60%, am bevorzugtesten höher als 75%.

Die spezifische Aktivität (Ristocetin-Cofaktor-Aktivität pro mg Gesamtprotein) kann durch die Durchlaufchromatographie um wenigstens 100%, vorzugsweise um wenigstens 150%, am bevorzugtesten um wenigstens 200% erhöht werden.

Für eine Feinreinigung kann VWF an eine Hydroxylapatit-Matrix gebunden und anschließend eluiert. Diese Anwendungsform wird als "bindende Chromatographie" bezeichnet. Üblicherweise umfasst die bindende Chromatographie, dass
(a) VWF an die Hydroxylapatit-Matrix gebunden wird,
(b) Verunreinigungen bei niedrigerer Salzkonzentration herausgewaschen werden und
(c) anschließend die VWF-haltige Wertfraktion bei höherer Salzkonzentration eluiert wird.

In Schritt (a) wird eine Lösung, die VWF und ein oder mehrere verunreinigende Proteine enthält, mit der Hydroxylapatit-Matrix in Kontakt gebracht. Die Gesamtkonzentration an Natrium- und/oder Kaliumphosphat in dieser Lösung ist üblicherweise 0 bis 200 mM, vorzugsweise 1 bis 100 mM, bevorzugter 1 bis 50 mM, am bevorzugtesten 10 bis 30 mM.

In dem Waschschritt (b) wird die Hydroxylapatit-Matrix mit einem Puffer niedriger Salzkonzentration gewaschen. Die Gesamtkonzentration an Natrium- und/oder Kaliumphosphat in diesem Waschpuffer ist in der Regel 100 bis 300 mM, vorzugsweise 150 bis 250 mM, am bevorzugtesten 180 bis 240 mM.

In Schritt (c) kann die VWF-haltige Wertfraktion mit einem Puffer höherer Salzkonzentration eluiert werden. Der Elutionspuffer enthält in der Regel 200 bis 500 mM, vorzugsweise 250 bis 400 mM Natrium- und/oder Kaliumphosphat.

Durch Verschiebung der Salzkonzentrationen können Ausbeute und Reinheit verändert werden. Je höher die Salzkonzentration im Waschpuffer ist, desto sauberer ist die erhaltene Wertfraktion. Die Ausbeute erniedrigt sich dadurch allerdings. Weiterhin beeinflusst der gewählte pH-Wert die optimale Salzkonzentration für den Waschpuffer. Je niedriger der pH-Wert ist, desto stärker ist die Bindung von VWF an die Hydroxylapatit-Matrix. Entsprechend können die gewählten Salzkonzentrationen bei niedrigen pH-Werten höher sein, bei höheren pH-Werten hingegen niedriger.

Die bindende Hydroxylapatit-Chromatographie wird bei einem pH-Wert von 5 bis 7,5, vorzugsweise von 5,5 bis unter 6,8, am bevorzugtesten von 6,0 bis 6,5 durchgeführt. Üblicherweise haben Lauf-, Wasch- und Elutionspuffer, sowie die aufzutragende Proteinlösung den gleichen pH-Wert. Es sind aber auch Varianten praktikabel, bei denen diese Lösungen unterschiedliche pH-Werte aufweisen.

In dieser Form wird die VWF-haltige Lösung, z.B. eine vorgereinigte Kryopräzipitatlösung, bei einer niedrigen Salzkonzentration, vorzugsweise 0 - 100 mM Kalium- oder Natriumphosphat, besonders bevorzugt bei 10 - 30 mM, bei einem pH-Wert von 5,5 - 6,8, bevorzugt 6,0 - 6,5, auf eine Hydroxylapatit-Säule, z.B. keramisches Hydroxylapatit Typ 2 aufgetragen. Der Großteil der VWF-Moleküle wird unter diesen Bedingungen gebunden. Durch Waschen mit einer Lösungen mit höherer Salzkonzentration mit z.B. Kalium- oder Natriumphosphat, beispielsweise 230 mM Natriumphosphat pH 6,0, können Verunreinigungen, z.B. Fibronektin, ausgewaschen werden. Die Wertfraktion wird anschließend mit hochkonzentrierten Salzlösungen, z.B. Phosphatlösungen, wie z.B. 400 mM Natriumphosphat pH 6,0, eluiert.

Durch die bindende Chromatographie können aus den durch die Durchlaufchromatographie gereinigten VWF-Fraktionen VWF-Präparationen erhalten werden, die praktisch keine nachweisbaren Mengen an Fibrinogen und Fibronektin mehr enthalten. Wenn die Auftragslösung, die mit der Hydroxylapatit-Matrix in Kontakt gebracht wird, eine Plasmafraktion ist und/oder Fibrinogen oder Fibronektin enthält, kann eine praktisch quantitative Entfernung der verunreinigenden Proteine Fibrinogen und Fibronektin aus der Lösung erzielt werden. So ist die Konzentration an Fibrinogen in der Elutionsfraktion vorzugsweise niedriger als 25% der Konzentration an Fibrinogen in der Auftragslösung (vor der bindenden Chromatographie). Die Konzentration an Fibronektin in der Elutionsfraktion ist vorzugsweise niedriger als 10%, bevorzugter niedriger als 5% der Konzentration an Fibronektin in der Auftragslösung (vor der bindenden Chromatographie). Die Konzentrationen an Fibrinogen bzw. Fibronektin in der Elutionsfraktion (Wertfraktion) sind in der Regel unterhalb der Nachweisgrenze von ca. 1 µg/ml.

Durch eine im Anschluss an die erfindungsgemäße Durchlaufchromatographie durchgeführte bindende Chromatographie können VWF-Präparationen mit hoher spezifischer Aktivität erhalten werden. Die spezifische Aktivität in der Elutionsfraktion kann höher als 50 E/mg Protein sein, bevorzugt ist sie höher als 75 E/mg Protein, bevorzugter höher als 85 E/mg Protein, am bevorzugtesten wenigstens 100 E/mg Protein. Die VWF-Aktivität wird bestimmt mit dem Ristocetin-Cofaktor-Assay, der die Bindungsfähigkeit von VWF an den Plättchenrezeptor Glycoprotein Ib/IX unter dem Einfluss des Antibiotikums Ristocetin ermittelt. Die spezifische VWF-Aktivität kann bestimmt werden wie in den Beispielen beschrieben.

Für die Herstellung eines besonders reinen VWF-Präparates können die erfindungsgemäße Hydroxylapatit-Durchlaufchromatographie und die bindende Hydroxylaptatit-Chromatographie miteinander oder mit anderen Reinigungstechniken kombiniert werden. Als besonders geeignet hat sich nämlich gezeigt, zunächst nach dem oben beschriebenen Verfahren eine Durchlaufchromatographie mit Hydroxylapatit zur Abreicherung der Hauptverunreinigungen durchzuführen. Anschließend wird die Wertfraktion beispielsweise mit 1 M HCl auf pH 6,0 titriert. Die Probe wird, wie für die bindende Chromatographie beschrieben, auf eine Hydroxylapatit-Säule aufgetragen. VWF-Moleküle werden gebunden und selektiv eluiert.

In einer besonderen Ausführungsform wird daher zunächst eine Durchlaufchromatographie mit Hydroxylapatit durchgeführt, wobei VWF nicht an die Hydroxylapatit-Matrix bindet, und anschließend die Durchlauffraktion unter bindenden Bedingungen rechromatographiert und die VWF-Fraktion eluiert.

Die Schrittausbeuten liegen zwischen 65% und 85%, was im Hinblick auf die hohe Reinigungseffektivität sehr gut ist. Für die Durchlaufchromatographie ist es sinnvoll, aber nicht notwendig, Phosphationen als Puffersubstanz zu verwenden. Für die Elution von VWF bei der bindenden Chromatographie ist Phosphat ein spezifisches Agens.

In einer besonderen Ausführungsform kann anstelle von reinem Hydroxylapatit als Chromatographiematrix Fluoroapatit verwendet werden. Fluoroapatit wird durch Umsetzung von Hydroxylapatit mit einer fluoridhaltigen Substanz hergestellt. Die Firma Bio-Rad (München, Deutschland) erzeugt z.B. keramisches Fluoroapatit durch eine 90%-ige Umsetzung von keramischem Hydroxylapatit mit einem Fluorinreagenz. Fluoropatit ist im Vergleich zu Hydroxylapatit unter sauren pH-Wertbedingungen deutlich stabiler. Deshalb wird Fluoroapatit üblicherweise eingesetzt, um Chromatographien bei niedrigerem pH durchzuführen, als für Hydroxylapatit technisch sinnvoll. Für eine Aufreinigung von VWF-Molekülen mit Fluoroapatit kann die Chromatographie ähnlich zu dem für Hydroxylapatit vorgeschlagenen Verfahren durchgeführt werden. Salzkonzentrationen müssen dem gewählten pH-Wert (>= 5,0) angepasst werden.

Das erfindungsgemäße Verfahren kann weiterhin einen oder mehrere der folgenden Schritte umfassen:
(1) Schockgefrieren bei einer Temperatur von unter -30°C und Auftauen nahe 0°C (Kryopräzipitation)
(2) Ethanolfällung oder Adsorption an Aluminiumhydroxid
(3) Virusinaktivierung der VWF enthaltenden Zusammensetzung durch Solvens/Detergens-Behandlung
(4) Anionenaustauchchromatographie
(5) Fällung von Fibronektin durch Einstellung eines pH-Werts von unter pH 5,4
(6) Affinitätschromatographie
(7) Diafiltration oder Ultrafiltration
(8) Umpuffern durch Dialyse oder Gelfiltration
(9) Sterilfiltration
(10) Lyophilisierung
(11) Virusinaktivierung durch Hitzebehandlung (z.B. ca. 30 min bei ca. 100°C)

Die Verfahrensschritte (1) bis (5) werden vorzugsweise vor der Hydroxylapatit-Chromatographie durchgeführt. Es können aber auch weniger als die 5 Verfahrensschritte durchgeführt werden. Die Reihenfolge der Schritte ist nicht zwingend.

Die Schritte (6) bis (11) können durchgeführt werden, falls gewünscht. Insbesondere die Affinitätschromatographie ist aber nicht notwendig, da bereits durch die Hydroxylapatitchromatographie eine hohe Reinheit erreicht werden kann.

Als Ausgangsmaterial für die Verfahren der vorliegenden Erfindung kann somit eine vorgereinigte Plasmafraktion verwendet werden. Hierbei kann es sich um eine weiter aufgereinigte Kryopräzipitat-Lösung handeln. Die Kryopräzipitat-Lösung kann beispielsweise mit Aluminiumhydroxid gefällt werden und/oder chromatographisch weiter aufgereinigt werden. So kann die Kryopräzipitat-Lösung z. B. mit Aluminiumhydroxid gefällt werden und anschließend mittels Anionenaustauschchromatographie weiter aufgereinigt werden. Es ist ebenfalls bevorzugt, dass die Kryopräzipitat-Lösung einer Virusinaktivierung unterworfen wird. Bevorzugtes Verfahren zur Virusinaktivierung ist eine Solvens/DetergensBehandlung, wie sie im US-Patent Nr. 4,540,573 beschrieben ist.

Als Ausgangsmaterial kann ebenso eine Proteinlösung enthaltend rekombinanten VWF (rVWF) aus Zellkulturüberständen verwendet werden. Der Ausdruck "rVWF" umfasst auch Varianten mit einer gegenüber Wildtyp-VWF veränderten Aminosäuresequenz, wobei ein oder mehrere Aminosäuren subsituiert, deletiert und/oder hinzugefügt sein können. Die Varianten weisen in der Regel VWF-Aktivität auf. Verfahren zur Herstellung geeigneter Expressionsvektoren, zur Einschleusung der Vektoren in die Wirtszellen und zur Kultivierung der Wirtszellen sind dem Fachmann an sich bekannt (Fischer et al. Structural analysis of recombinant von Willebrand factor: identification of hetero and homo-dimers. FEBS Lett 1994; 351:345-348. Fischer et al. Structural analysis of recombinant von Willebrand factor produced at industrial scale fermentation of transformed CHO cells coexpressing recombinant furin. FEBS Lett 1995; 375:259-262).

Insbesondere bei Verwendung von Plasmafraktionen kann vor der Hydroxylapatit-Chromatographie eine pH-Fällung zur Abtrennung von Fibronektin durchgeführt werden. Die pH-Fällung zur Abtrennung von Fibronektin aus einer Plasmafraktion umfasst beispielsweise, dass man
(i) den pH-Wert der Plasmafraktion auf unter pH 5,4 einstellt, so dass sich ein Niederschlag bildet und
(ii) den gebildeten Niederschlag abtrennt.

Der Ausdruck "Plasmafraktion" bezeichnet in diesem Zusammenhang eine Zusammensetzung, die aus Plasma erhalten wurde und verschiedene Plasmaproteine enthält. Die Plasmafraktion, die als Ausgangszusammensetzung in Schritt (i) eingesetzt wird, ist eine flüssige Zusammensetzung. Vorzugsweise ist die flüssige Zusammensetzung eine Lösung oder eine Suspension, am bevorzugtesten ist die Zusammensetzung eine Lösung. In einer besonderen Ausführungsform ist die Plasmafraktion gelöstes Kryopräzipitat. Dieses gelöste Kryopräzipitat kann durch verschiedene Verfahren vorgereinigt sein. Beispiele sind Aluminiumhydroxid-Behandlung, Solvens-/Detergens-Behandlung und/oder Anionenaustauschchromatographie. Die Konzentration an Natriumchlorid oder Kaliumchlorid in der Plasmafraktion ist vorzugsweise 50 bis 250 mM, bevorzugter 100 bis 200 mM, am bevorzugtesten 120 bis 150 mM. Die Plasmafraktion kann beispielsweise folgende Puffersubstanzen enthalten: Citrationen, Acetationen, Phosphationen und/oder Aminosäuren.

Die Konzentration an Fibronektin in der Plasmafraktion, die Schritt (i) unterworfen wird, ist in der Regel mindestens 0,05 g/l, vorzugsweise wenigstens 0,1 g/l, noch bevorzugter wenigstens 0,25 g/l, am bevorzugtesten wenigstens 0,5 g/l. Die Konzentration an Fibronektin in der Plasmafraktion kann beispielsweise 0,1 bis 5 g/l, vorzugsweise 0,1 bis 2 g/l sein.

Zur Abtrennung von Fibronektin aus der Plasmafraktion wird der pH-Wert der Plasmafraktion auf unter pH 5,4 eingestellt. Dabei bildet sich ein Niederschlag, der Fibronektin enthält. Vorzugsweise wird der pH-Wert auf unter pH 5,3 eingestellt, noch bevorzugter auf unter pH 5,2. Der eingestellte pH-Wert liegt somit vorzugsweise in einem Bereich von pH 4,5 bis unter 5,4, bevorzugt in einem Bereich von pH 4,7 bis 5,3, bevorzugter in einem Bereich von pH 4,8 bis 5,2, noch bevorzugter in einem Bereich von pH 4,9 bis 5,1. In der Regel wird das Einstellen des pH-Werts durch Zugabe einer sauren Komponente erreicht. Als saure Komponente können verschiedene Säuren eingesetzt werden, beispielsweise Salzsäure, Phosphorsäure oder Essigsäure. Die saure Komponente wird üblicherweise über einen bestimmten Zeitraum zugegeben, beispielsweise tropfenweise. Somit wird nach und nach ein pH-Wert im oben näher definierten Bereich eingestellt ("titriert").

Während und nach dem Einstellen des pH-Werts wird die Plasmafraktion vorzugsweise in Bewegung gehalten oder durchmischt, beispielsweise durch Rühren. Es ist weiterhin bevorzugt, dass nach Einstellen des pH-Werts die Plasmafraktion für einen bestimmten Zeitraum weiter durchmischt wird (z.B. durch Rühren), im allgemeinen für wenigstens 10 Minuten, vorzugsweise für wenigstens 20 Minuten, am bevorzugtesten für einen Zeitraum von 30 bis 90 Minuten. In diesem Zeitraum bilden sich klebrige Aggregate, die zu einem erheblichen Anteil Fibronektin erhalten. Daher ist es gemäß einer bevorzugten Ausführungsform vorgesehen, dass ein geeigneter Rührer, z.B. ein Anker- oder Paddelrührer eingesetzt wird, an dessen Rührblatt sich der Niederschlag anheftet. Das präzipitierte Fibronektin kann somit leicht der Lösung entzogen werden.

Die pH-Fällung zur Abtrennung von Fibronektin kann in einem breiten Temperaturspektrum durchgeführt werden, z.B. von ca. 1°C bis ca. 37°C. Bevorzugte Temperaturbereiche sind 4 bis 35°C, bevorzugter 10 bis 30°C, am bevorzugtesten wird das Verfahren bei 20 bis 25°C durchgeführt.

Durch die pH-Fällung zur Abtrennung von Fibronektin aus Plasmafraktionen kann die Konzentration an Fibronektin in der Plasmafraktion um wenigstens 50% verringert werden. Vorzugsweise wird die Konzentration an Fibronektin in der Plasmafraktion um 70 bis 99%, bevorzugter um 80 bis 99%, am bevorzugtesten um 90 bis 98% oder um 95 bis 98% verringert. In einer besonderen Ausführungsform liegt der Verlust an VWF in dem Fällungsschritt bei höchstens 50%, bevorzugt bei höchstens 40%, bevorzugter bei höchstens 30%, noch bevorzugter bei höchstens 20%, am bevorzugtesten bei höchstens 10%.

Ein weiterer Aspekt der vorliegenden Offenbarung ist eine VWF enthaltende Zusammensetzung erhältlich durch ein erfindungsgemäßes Verfahren, wie es in dieser Anmeldung beschrieben ist. Vorzugsweise handelt es sich um eine im wesentlichen reine VWF-Präparation. "Im wesentlichen rein" bedeutet, dass der Proteinanteil der Zusammensetzung mindestens 70% VWF-Moleküle enthält.

Durch das erfindungsgemäße Verfahren können ohne Feinreinigung spezifische Aktivitäten von > 60 E pro mg Protein und mit Feinreinigung von >100 E pro mg Protein erzielt werden. Die Proteinlösung kann anschließend umgepuffert und lyophilisiert werden. Optional sind auch weitere Virusinaktivierungsschritte, wie z.B. die Inkubation des Lyophilisats bei 100°C für 30 Minuten, möglich.

Hydroxylapatit, insbesondere in seiner keramikstabilisierten Form ist außerordentlich gut geeignet, um Prozesse im Industriemaßstab durchzuführen. Es ist beispielsweise für die Abtrennung von Fibronektin von VWF-haltigen Plasmafraktionen wesentlich kosteneffektiver und reproduzierbarer einzusetzen, als z.B. Gelatine-Sepharose oder andere Affinitätschromatographie-Medien. Aufgrund seiner Trenneigenschaften bietet Hydroxylapatit eine bessere Auflösung als die vielfach beschriebenen lonenaustauschchromatographiemedien. Bei dem Hydroxylapatit-Reinigungsverfahren müssen den Puffern weder Calcium noch Aminosäuren zugesetzt werden.

Da Hydroxylapatit wesentlich höhere Flussraten und längere Standzeiten als häufig verwendete Affinitätschromatographie-Medien zulässt, wird mit diesem Verfahren ein äußerst wirtschaftliches Herstellverfahren für ein hochreines VWF-Präparat zur Verfügung gestellt. Die hervorragende Sanitisierbarkeit des Materials mit z.B. 1 M NaOH führt darüber hinaus zu einer optimalen Produktsicherheit.

Die verschiedenen in dieser Anmeldung beschriebenen Ausführungsformen können miteinander kombiniert werden.

Die nachfolgenden Beispiele erläutern die Erfindung näher.

### Beispiel 1: Reinigung einer VWF-haltigen Plasmafraktion mittels Hydroxylapatit-Durchlaufchromatographie

Die VWF-haltige Proteinlösung durchlief folgende Vorreinigungsstufen, wie sie, z.B. in WO 9315105 A1 beschrieben sind: Eine Kryopräzipitat-Lösung wurde einer Aluminiumhydroxid-Fällung unterzogen. Anschließend wurde eine Virusinaktivierung mittels S/D-Behandlung durchgeführt. Bei der folgenden Anionenaustauschchromatographie fiel eine VWF-haltige Waschfraktion an, die vor allem mit Fibrinogen und Fibronektin verunreinigt war. Die Proteinlösung wurde durch Titration auf pH 5.2 einem Fällungsschritt unterzogen, bei dem ein großer Teil des Fibronektins und Fibrinogens entfernt wurde. Anschließend wurde eine Ultra- und Diafiltration durchgeführt, wobei die Proteinlösung ca. 7-fach ankonzentriert und gegen den Laufpuffer der folgenden Chromatographie diafiltriert wurde. Die so erhaltene Proteinlösung enthielt ca. 930 µg/ml VWF-Antigen, 270 µg/ml Fibrinogen-Antigen und 2400 µg/ml Fibronektin-Antigen. Die Proteinlösung wurde auf eine in 10 mM Na-Phosphat pH 7,0 equilibrierte Hydroxylapatit-Säule (CHT Typ 1, Bio-Rad, München, Deutschland) aufgetragen. Die Durchlauffraktion enthielt ca. 560 µg/ml VWF-Antigen, 5 µg/ml Fibrinogen-Antigen und 50 µg/ml Fibronektin-Antigen. Die Massenbilanz ergibt eine Schrittausbeute von 78% für das VWF-Antigen. Aufgrund der sehr guten Reinigungseffektivität ist die Ausbeute als hervorragend anzusehen.

**Tabelle 2: Reinigung einer VWF-haltigen Plasmafraktion mittels Hydroxylapatit-Durchlaufchromatographie**

| | VWF-Antigen | Fibrinogen-Antigen | Fibronektin-Antigen | Ausbeute |
|---|---|---|---|---|
| Ausgang | 930 µg/ml | 270 µg/ml | 2400 µg/ml | |
| Durchlauffraktion | 560 µg/ml | 5 µg/ml | 50 µg/ml | 78% |

Die Konzentration an VWF-Antigen wurde mit Hilfe des STA^{®} Compact der Firma Diagnostica Stago (Roche Diagnostics, Mannheim) und deren Test-Reagenzien (STA LIA vWF) bestimmt.

Für die Bestimmung der Menge an Fibrinogen-Antigen und Fibronektin-Antigen wurden nephelometrische Methoden zur quantitativen Bestimmung der Fibrinogen-Antigen- und Fibrinogen-Antigen-Konzentration im Beckman-Array^{®} 360 (Beckman Coulter, Monheim) verwendet.

### Beispiel 2: Feinreinigung unter Verwendung einer bindenden Hydroxylapatit-Chromatographie

Eine nach der in Beispiel 1 hergestellten Proteinlösung wurde zur weiteren Aufreinigung auf pH 6,0 titriert. Anschließend wurde die Lösung auf eine Hydroxylapatit-Säule (CHT Typ 1, Bio-Rad, München, Deutschland) aufgetragen, die im Laufpuffer (20 mM Natriumphosphat pH 6,0) equilibriert worden war. Der VWF wurde quantitativ gebunden. Eine erste Elution wurde mit 230 mM Natriumphosphat pH 6,0 durchgeführt, die zweite mit 400 mM Natriumphosphat pH 6,0. Die aufgetragene Proteinlösung enthielt 540 µg/ml VWF-Antigen, 4 µg/ml Fibrinogen und 48 µg/ml Fibronektin. Die erste Elutionsfraktion enthielt 38 µg/ml VWF-Antigen, 2 µg/ml Fibronektin-Antigen. Die Fibrinogen-Antigen-Konzentration lag unterhalb des Detektionslimits von 1 µg/ml. Die Wertfraktion (2. Elutionsfraktion) wies eine VWF-Antigen-Konzentration von 173 µg/ml auf. Weder Fibrinogen-Antigen, noch Fibronektin-Antigen konnte detektiert werde. Die VWF-Aktivität betrug 23 E/ml, die Gesamtproteinkonzentration (OD₂₈₀ₙₘ) 0,23 mg/ml. Es wurde eine spezifische Aktivität von 100 E / mg Protein erzielt.

**Tabelle 3: Feinreinigung unter Verwendung einer bindenden Hydroxylapatit-Chromatographie**

| | VWF-Antigen | Fibrinogen-Antigen | Fibronektin-Antigen | Spezifische Aktivität |
|---|---|---|---|---|
| Ausgang | 540 µg/ml | 4 µg/ml | 48 µg/ml | 56 E/mg |
| Elutionsfraktion 1 | 38 µg/ml | n.d. | 2 µg/ml | 12 E/mg |
| Elutionsfraktion 2 | 173 µg/ml | n.d. | n.d. | 100 E/mg |

Die VWF-Aktivität wurde als Ristocetin-Cofaktor-Aktivität über Plättchenagglutination mit dem BCT^{®}-Anaiyser (Behring Coagulation Timer, Dade Behring, Schwalbach) bestimmt.

Die Proteinbestimmung wurde nach dem Lambert-Beerschen Gesetz (A=ε·c·d) durchgeführt, wobei
A=Absorption bei 280 nm
ε=Absorptionskoeffizient (hier theoretischer Absorptionskoeffizient 0,75 cm²/mg)
c=Proteinkonzentration in mg/ml
d=Schichtdicke in cm.

### Beispiel 3: Erhöhung der spezifischen Aktivität einer vorgereinigten VWF-haltigen Plasmafraktion mittels bindender Fluoroapatit-Chromatographie

Die Ausgangslösung wurde entsprechend Beispiel 1 präpariert. Die Proteinlösung enthielt bei einer Proteinkonzentration von 0,83 mg/ml eine spezifische Aktivität von 64,8 E/mg. Durch Zugabe von HCl wurde die Proteinlösung auf einen pH-Wert von 6,0 titriert. Anschließend wurde die Lösung auf eine Fluoroapatit-Säule (CFT Typ 1, Bio-Rad, München, Deutschland) mit einem Gelbettvolumen von 13,2 ml aufgetragen, die im Laufpuffer (20 mM Na-Phosphat pH 6,0) equilibriert worden war. Ein Teil des VWF wurde gebunden. Eine Abtrennung von Verunreinigungen wurde durch eine erste Elution mit einem Puffer erzielt, der 241 mM Na-Phosphat pH 6,0 enthielt Elutionsfraktion 1). Die Wertfraktion wurde im Anschluss mit 400 mM Na-Phosphat pH 6,0 eluiert. Durch diesen chromatographischen Schritt konnte die spezifische Aktivität auf 77,0 E / mg Protein erhöht werden. Aufgrund der schwächeren Bindung von VWF bei den gewählten pH-Bedingungen findet keine komplette Bindung des VWF statt, so dass sich ein Teil in der Durchlauffraktion befindet.

**Tabelle 4: Erhöhung der spezifischen VWF-Aktivität einer vorgereinigten VWF-haltigen Plasmafraktion mittels Fluoroapatit-Chromatographie**

| | Spezifische Aktivität [E pro mg Protein] |
|---|---|
| Ausgang | 64,8 |
| Durchlauf | 47,1 |
| Elutionsfraktion 1 | 39,3 |
| Wertfraktion | 77,0 |

### Ergänzende Literaturstellen

Folgende Literaturstellen zu verschiedenen Analytikmethoden werden ergänzend genannt:
**VWF-Aktivität:**
   Veyradier A, Fressinaud E, Meyer D (1998): Laboratory diagnosis of von Willebrand disease. Int J Lab Res 28 (4): 201-210.
**VWF-Antigen:**
   Budde U, et al. (1984): Acquired von Willebrand's disease in the myeloproliferative syndrome. Blood 64 (5): 981-985.
   Newman DJ, Henneberry H, Price CP (1992): Particle enhanced light scattering immunoassay. Ann Clin Biochem 29 (Pt1): 22-42.
**Fibronektin-Antigen:**
   Sandberg L, et al. (1985): Plasma fibronectin levels in acute and recovering malnourished children. Clin Physiol Biochem. 3(5):257-264.
   Colli A, et al. (1986): Diagnostic accuracy of fibronectin in the differential diagnosis of ascites. Cancer.: 58(11):2489-2493.
**Fibrinogen-Antigen:**
   Ernst E, Resch KL (1993): Fibrinogen as a cardiovascular risk factor: a meta-analysis and review of the literature. Ann Intern Med.: 118(12):956-963.
   Jelic-Ivanovic Z, Pevcevic N (1990): Fibrinogen determination by five methods in patients receiving streptokinase therapy. Clin Chem.: 36(4):698-699.

## Patentansprüche

1. Verfahren zur Aufreinigung von VWF, **dadurch gekennzeichnet, dass**
(i) eine Zusammensetzung, die VWF und ein oder mehrere verunreinigende Proteine enthält, mit einer Hydroxylapatit-Matrix in Kontakt gebracht wird, so dass wenigstens ein verunreinigendes Protein an die Hydroxylapatit-Matrix gebunden wird, während VWF im wesentlichen nicht an die Hydroxylapatit-Matrix gebunden wird, und gegebenenfalls
(ii) ungebundenes VWF von der Hydroxylapatit-Matrix getrennt wird.

2. Verfahren nach Anspruch 1, **dadurch gekennzeichnet, dass** VWF sich im Durchlauf befindet und wenigstens ein verunreinigendes Protein an das Hydroxylapatit gebunden wird.

3. Verfahren nach einem der Ansprüche 1 bis 2, **dadurch gekennzeichnet, dass** das verunreinigende Protein Fibronektin oder Fibrinogen ist.

4. Verfahren nach einem der Ansprüche 1 bis 3, **dadurch gekennzeichnet, dass** die Hydroxylapatit-Chromatographie bei einem pH-Wert von 6,5 bis 8,0, vorzugsweise von 6,8 bis 7,5 durchgeführt wird.

5. Verfahren nach einem der Ansprüche 1 bis 4, **dadurch gekennzeichnet, dass** als Laufpuffer eine Lösung enthaltend Natriumphosphat und/oder Kaliumphosphat verwendet wird.

6. Verfahren nach Anspruch 1 bis 5, **dadurch gekennzeichnet, dass** VWF in einem weiteren chromatographischen Schritt an eine Hydroxylapatit-Matrix gebunden wird und anschließend eluiert wird.

7. Verfahren nach Anspruch 6, **dadurch gekennzeichnet, dass** in dem weiteren chromatographischen Schritt
(a) VWF an die Hydroxylapatit-Matrix gebunden wird,
(b) Verunreinigungen herausgewaschen werden und
(c) anschließend die VWF-haltige Wertfraktion bei höherer Salzkonzentration eluiert wird.

8. Verfahren nach Anspruch 7, **dadurch gekennzeichnet dass** in Schritt (a) eine Zusammensetzung, die VWF, ein oder mehrere verunreinigende Proteine und 1 bis 200 mM, vorzugsweise 1 bis 50 mM Natrium- und/oder Kaliumphosphat enthält, mit der Hydroxylapatit-Matrix in Kontakt gebracht wird.

9. Verfahren nach Anspruch 7 oder 8, **dadurch gekennzeichnet, dass** in Schritt (b) die Hydroxylapatit-Matrix mit einem Puffer enthaltend 100 bis 300 mM, vorzugsweise 150 bis 250 mM Natrium- und/oder Kaliumphosphat gewaschen wird.

10. Verfahren nach einem der Ansprüche 7 bis 9, **dadurch gekennzeichnet, dass** in Schritt (c) die VWF-haltige Wertfraktion mit einem Puffer, der 200 bis 500 mM, vorzugsweise 250 bis 400 mM Natrium- und/oder Kaliumphosphat enthält, eluiert wird.

11. Verfahren nach einem der Ansprüche 6 bis 10, **dadurch gekennzeichnet, dass** die Hydroxylapatit-Chromatographie bei einem pH-Wert von 5 bis 7,5, vorzugsweise von 5,5 bis unter 6,8 durchgeführt wird.

12. Verfahren nach einem der Ansprüche 1 bis 11, **dadurch gekennzeichnet, dass** zunächst eine Durchlaufchromatographie mit Hydroxylapatit durchgeführt wird, wobei VWF nicht an die Hydroxylapatit-Matrix bindet, und anschließend die Durchlauffraktion unter bindenden Bedingungen rechromatographiert und die VWF-Fraktion eluiert wird.

13. Verfahren nach einem der Ansprüche 1 bis 12, **dadurch gekennzeichnet, dass** als Ausgangsmaterial eine vorgereinigte Plasmafraktion verwendet wird.

14. Verfahren nach einem der Ansprüche 1 bis 13, **dadurch gekennzeichnet, dass** als Ausgangsmaterial eine weiter aufgereinigte Kryopräzipitat-Lösung verwendet wird.

15. Verfahren nach einem der Ansprüche 1 bis 14, **dadurch gekennzeichnet, dass** als Ausgangsmaterial eine mit Aluminiumhydroxid gefällte Kryopräzipitat-Lösung verwendet wird.

16. Verfahren nach einem der Ansprüche 1 bis 15, **dadurch gekennzeichnet, dass** als Ausgangsmaterial eine chromatographisch vorgereinigte Aluminiumhydroxid-gefällte Kryopräzipitat-Lösung verwendet wird.

17. Verfahren nach einem der Ansprüche 1 bis 16, **dadurch gekennzeichnet, dass** vor der Hydroxylapatit-Chromatographie eine pH-Fällung zur Abtrennung von Fibronektin durchgeführt wird.

18. Verfahren nach einem der Ansprüche 1 bis 17, **dadurch gekennzeichnet, dass** als Ausgangsmaterial eine VWF-haltige Proteinlösung aus Zellkulturüberständen verwendet wird.

19. Verfahren nach einem der Ansprüche 1 bis 18, **dadurch gekennzeichnet, dass** Hydroxylapatit verwendet wird, das Fluoridionen enthält.

## Claims

1. A process for purifying VWF, **characterized in that**
(i) a composition containing VWF and one or more contaminating proteins is contacted with a hydroxylapatite matrix so as to bind at least one contaminating protein to the hydroxylapatite matrix, while VWF is substantially not bound to the hydroxylapatite matrix, and optionally
(ii) unbound VWF is separated from the hydroxylapatite matrix.

2. The process according to claim 1, **characterized in that** VWF is found in the flowthrough and at least one contaminating protein is bound to the hydroxylapatite.

3. The process according to claim 1 or 2, **characterized in that** the contaminating protein is fibronectin or fibrinogen.

4. The process according to any one of claims 1 to 3, **characterized in that** the hydroxylapatite chromatography is carried out at a pH of 6.5 to 8.0, preferably 6.8 to 7.5.

5. The process according to any one of claims 1 to 4, **characterized in that** a solution containing sodium phosphate and/or potassium phosphate is used as the running buffer.

6. The process according to claims 1 to 5, **characterized in that** VWF is bound to a hydroxylapatite matrix in a further chromatographic step and then eluted.

7. The process according to claim 6, **characterized in that** in the further chromatographic step
(a) VWF is bound to the hydroxylapatite matrix,
(b) impurities are washed out, and
(c) the VWF containing fraction of interest is then eluted at a higher salt concentration.

8. The process according to claim 7, **characterized in that** in step (a) a composition containing VWF, one or more contaminating proteins and 1 to 200 mM, preferably 1 to 50 mM, sodium and/or potassium phosphate is contacted with the hydroxylapatite matrix.

9. The process according to claim 7 or 8, **characterized in that** in step (b) the hydroxylapatite matrix is washed with a buffer containing 100 to 300 mM, preferably 150 to 250 mM, sodium and/or potassium phosphate.

10. The process according to any one of claims 7 to 9, **characterized in that** in step (c) the VWF containing fraction of interest is eluted with a buffer containing 200 to 500 mM, preferably 250 to 400 mM, sodium and/or potassium phosphate.

11. The process according to any one of claims 6 to 10, **characterized in that** the hydroxylapatite chromatography is carried out at a pH of 5 to 7.5, preferably 5.5 to below 6.8.

12. The process according to any one of claims 1 to 11, **characterized in that** a flowthrough chromatography with hydroxylapatite is initially carried out, wherein VWF does not bind to the hydroxylapatite matrix, and then the flowthrough fraction is re-chromatographed under binding conditions, and the VWF fraction is eluted.

13. The process according to any one of claims 1 to 12, **characterized in that** a previously purified plasma fraction is used as the starting material.

14. The process according to any one of claims 1 to 13, **characterized in that** a further purified cryoprecipitate solution is used as the starting material.

15. The process according to any one of claims 1 to 14, **characterized in that** a cryoprecipitate solution precipitated with aluminum hydroxide is used as the starting material.

16. The process according to any one of claims 1 to 15, **characterized in that** a chromatographically pre-purified cryoprecipitate solution precipitated with aluminum hydroxide is used as the starting material.

17. The process according to any one of claims 1 to 16, **characterized in that** a pH precipitation is carried out prior to the hydroxylapatite chromatography to separate fibronectin.

18. The process according to any one of claims 1 to 17, **characterized in that** a VWF containing protein solution from cell culture supernatants is used as the starting material.

19. The process according to any one of claims 1 to 18, **characterized in that** hydroxypalatite is used which contains fluoride ions.

## Revendications

1. Procédé de purification du FVW, **caractérisé en ce que**
(i) une composition qui contient le FVW et une ou plusieurs protéines contaminantes, est mise en contact avec une matrice d'hydroxylapatite de sorte qu'au moins une protéine contaminante se fixe à la matrice d'hydroxylapatite, tandis que le FVW ne se fixe substantiellement pas à la matrice d'hydroxylapatite, et éventuellement
(ii) le FVW non fixé est séparé de la matrice d'hydroxylapatite.

2. Procédé selon la revendication 1, **caractérisé en ce que** le FVW est circulant et au moins une protéine contaminante est fixée sur l'hydroxylapatite.

3. Procédé selon l'une des revendications 1 à 2, **caractérisé en ce que** la protéine contaminante est la fibronectine ou le fibrinogène.

4. Procédé selon l'une des revendications 1 à 3, **caractérisé en ce que** la chromatographie de l'hydroxylapatite est réalisée à un pH de 6,5 à 8,0, de préférence de 6,8 à 7,5.

5. Procédé selon l'une des revendications 1 à 4, **caractérisé en ce que** l'on utilise comme tampon d'élution, une solution contenant du phosphate de sodium et/ou du phosphate de potassium.

6. Procédé selon la revendication 1 à 5, **caractérisé en ce que**, dans une autre étape chromatographique, le FVW est fixé à une matrice d'hydroxylapatite et est ensuite élué.

7. Procédé selon la revendication 6, **caractérisé en ce que**, dans l'autre étape chromatographique
(a) le FVW est fixé à la matrice d'hydroxylapatite.
(b) les contaminants sont éliminés par lavage et
(c) ensuite, la fraction de valeur contenant le FVW est éluée à une concentration de sel supérieure

8. Procédé selon la revendication 7, **caractérisé en ce que**, à l'étape (a), une composition qui contient le FVW, une ou plusieurs protéines contaminantes et 1 à 200 mM, de préférence, 1 à 50 mM de phosphate de sodium et/ou de potassium, est mise en contact avec la matrice d'hydroxylapatite.

9. Procédé selon la revendication 7 ou 8, **caractérisé en ce que**, à l'étape (b), la matrice d'hydroxylapatite est lavée avec un tampon contenant 100 à 300 mM, de préférence 150 à 250 mM de phosphate de sodium et/ou de phosphate de potassium.

10. Procédé selon l'une des revendications 7 à 9, **caractérisé en ce que**, à l'étape (c), la fraction de valeur contenant le FVW est éluée avec un tampon qui contient 200 à 500 mM, de préférence 250 à 400 mM de phosphate de sodium et/ou de potassium.

11. Procédé selon l'une des revendications 6 à 10, **caractérisé en ce que** la chromatographie de l'hydroxylapatite est réalisée à un pH de 5 à 7,5, de préférence de 5,5 à moins de 6,8.

12. Procédé selon l'une des revendications 1 à 11, **caractérisé en ce que** tout d'abord, une chromatographie continue est réalisée avec l'hydroxylapatite, dans laquelle le FVW ne se fixe pas à la matrice d'hydroxylapatite et ensuite, la fraction continue est rechromatographiée dans des conditions de fixation et la fraction du FVW est éluée.

13. Procédé selon l'une des revendications 1 à 12, **caractérisé en ce que** l'on utilise comme matériau de départ, une fraction plasmatique pré-purifiée.

14. Procédé selon l'une des revendications 1 à 13, **caractérisé en ce que** l'on utilise comme matériau de départ, une autre solution de cryoprécipité purifiée.

15. Procédé selon l'une des revendications 1 à 14, **caractérisé en ce que** l'on utilise comme matériau de départ, une solution de cryoprécipité précipitée avec de l'hydroxyde d'aluminium.

16. Procédé selon l'une des revendications 1 à 15, **caractérisé en ce que** l'on utilise comme matériau de départ, une solution de cryoprécipité pré-purifiée, précipitée avec de l'hydroxyde d'aluminium.

17. Procédé selon l'une des revendications 1 à 16, **caractérisé en ce que**, avant la chromatographie de l'hydroxylapatite, une précipitation liée au pH est réalisée pour la séparation de la fibronectine.

18. Procédé selon l'une des revendications 1 à 17, **caractérisé en ce que** l'on utilise comme matériau de départ, une solution de protéine contenant le FVW de surnageants de culture cellulaire.

19. Procédé selon l'une des revendications 1 à 18, **caractérisé en ce que** l'on utilise l'hydroxylapatite qui contient des ions fluorure.
